# EUROPEAN PATENT SPECIFICATION

(11) **EP 3 727 663 B1**
(45) Date of publication and mention of the grant of the patent: **06.05.2026**
(21) Application number: 18892446.8
(22) Date of filing: 21.12.2018
(51) Int. Cl.: C09K 3/22, C01C 1/26, C07C 215/40

(54) **USE OF AQUEOUS SOLUTION OF ORGANIC AMMONIUM CARBOXYLATE IN PREVENTING DUSTING OF FINE MATERIAL AND COMBINATION OF AN AQUEOUS SOLUTION OF ORGANIC AMMONIUM CARBOXYLATE AND FINE MATERIAL**
VERWENDUNG EINER WÄSSRIGEN LÖSUNG VON ORGANISCHEM AMMONIUMCARBOXYLAT ZUR VERHINDERUNG DER STAUBBILDUNG VON FEINEM MATERIAL UND KOMBINATION EINER WÄSSRIGEN LÖSUNG VON ORGANISCHEM AMMONIUMCARBOXYLAT UND FEINEM MATERIAL
UTILISATION D'UNE SOLUTION AQUEUSE DE CARBOXYLATE D'AMMONIUM ORGANIQUE POUR EMPÊCHER LA FORMATION DE POUSSIÈRE DE MATÉRIAU FIN ET COMBINAISON D'UNE SOLUTION AQUEUSE DE CARBOXYLATE D'AMMONIUM ORGANIQUE ET DE MATÉRIAU FIN

(30) Priority: 22.12.2017 FI 20176149
(43) Date of publication of application: 28.10.2020
(73) Proprietor: Oy Granula Ab Ltd, 48101 Kotka (FI)
(72) Inventor: AHLNÄS, Thomas, FI-48130 Kotka (FI)
(74) Representative: Berggren Oy
(86) International application number: PCT/FI2018/050977
(87) International publication number: WO 2019/122537

(56) References cited:
- WO-A1-2011/056322
- WO-A1-2011/089322
- KR-B1- 101 606 738
- US-A- 2 195 573
- US-A- 4 254 166
- US-A1- 2012 111 583

## Description

### FIELD OF INVENTION

The invention relates to the method of preamble of claim 1.

The invention relates also to the combination of mist or drops of an aqueous solution of organic ammonium carboxylate of formula (I) and fine material of preamble of claim 21

The invention relates to method for spraying an aqueous solution of organic ammonium carboxylate in controlling dusting of sand, crushed stone, , crushed expanded clay, or crushed expanded clay aggregate, crushed cement or concrete, or chopped organic material. The organic ammonium carboxylate which is used as an aqueous solution is especially adapted for controlling dust formation in applications where the biodegradation and low BOD is also required. Preferably it is used the environmentally benign freezing point depressant compositions for preventing dust formation and for preventing ice formation (anti-icing) within the compositions itself.

### BACKGROUND OF INVENTION

Mineral dust and street dust (from street rubble) are serious health problems for example in mining industry and cities. There are also other applications wherein dusting of sand, fine crushed stone and soil will cause mineral dust which is a serious health problem. Dusting causes also damage to equipment and vehicle used for example in mining industry, these include vehicles, electric motors, transport bands etc. Specifically in mining industry mineral, dust will intrude itself into ore silos and transportation vehicles and in mining equipment causing freezing in winter time.

It has suggested several ways to reduce mineral dust in mining industry. One alternative is to bind mineral dust by means of aqueous or water-borne solutions. However, none of water-borne solutions have been successful this far.

If an aqueous solution is used in dust control, water have a tendency to evaporate after been sprayed onto surface of crushed stone, sand or soil. This requires usage of relatively big amounts of aqueous solution. Using plenty of water in binding dust will then cause other problems, such as mineral puddling.

One important aspect when using aqueous solutions in controlling mineral dust is possible disturbances brought into mining industry processes alongside with said aqueous solution: especially chlorides of potassium, magnesium, calcium and sodium have a tendency to cause disturbances in ore recovery processes, for example in extraction stages.

One important aspect is also biodegradativity of solutions used for controlling mineral dust.

Additionally using aqueous solutions to dust binding may also cause freezing of aqueous solution itself. Freezing point depressant compositions are used wide-spreadly for variety of purposes, especially for lowering freezing point of an aqueous system so that ice cannot be formed on surfaces or within the aqueous systems or for melting ice formed in those aqueous systems. However dust control with freezing point depressant compositions are relative rare because the effectiveness of the freezing point depressant compositions depends on the molar freezing point lowering effect, the number of ionic species that are made available and to the degree to which the compositions can be dispersed in the liquid phase.

Most freezing point depressant compositions are either based on salts such as sodium chloride or potassium formate or alcohols such as glycols and methanol. Alkali and alkali-earth metal salts of carboxylic acid such as potassium formate, sodium formate, potassium acetate and sodium acetate have found increasing use in the area of deicing mainly due to their low environment impact and low viscosity at minus temperatures. However, using these compositions for controlling dusting in mining industry is not a viable option, because potassium, natrium, magnesium and calcium chlorides may interfere ore recovering process.

### GENERAL DESCRIPTION OF THE INVENTION

The above prior art as a starting point, the objection of the present invention was to solve or at least to alleviate above mentioned problems.

Thus, the general objection of the present invention was to provide an aqueous solution which could be used as a combined freezing point depressant and an aqueous solution for controlling dusting and which is also environmentally benign and which do not form chlorides which may interfere mining process.

The ideal dusting control agent and freezing point depressant composition adapted to use for mining industry would have following properties:
- it should prevent effectively formation of mineral dust from grounded stones, sand and soil,
- it should be free of mining process disturbing halides such as chlorides of alkali and alkali-earth metals especially chlorides of potassium, sodium, magnesium and calcium,
- it should have relatively low biological (BOD) and chemical oxygen demand (COD),
- it should be effective at low temperatures, i.e. it should have low viscosity and low freezing point,
- it should not cause mineral puddling, that is, it should be effective when used also as relatively small amounts.

The present inventors have surprisingly found that the ideal solution for above mentioned problems relating to reducing mineral dust in air with an aqueous solution and in the same time lowering the freezing point of said aqueous solution itself is to use specific aqueous solution of organic ammonium carboxylate of formula (I), as defined in claim 1:

[NR¹R²R³R⁴]⁺ [R⁵(COO)], (I),

in which R¹, R², and R³ are selected from the group composing of hydrogen and methyl, R⁴ is a C₁-C₄-alkyl substituted with a hydroxyl group, R⁵ is hydrogen or methyl ,
as a mist or drops in preventing dusting of fine material and in lowering the freezing point of said aqueous solution on the surface of said fine material.

Organic ammonium carboxylate stands for a salt or a complex formed of an ammonium cation and a carboxylic anion. Hence one or more ammonium ions of the salt or complex may be primary (RNH₃⁺), secondary (R₂NH₂⁺), tertiary (R₃NH⁺) or quaternary (R₄N⁺). The carboxylate ion of the salt or complex may be monovalent (RCOO⁻) or polyvalent (R(COO⁻)_{n>1}), and in that case it may also comprise unneutralised carboxyl groups (-COOH). In the latter case, R⁵ is defined as being substituted with carboxyl.

Since the group R⁵ is associated with a carboxylate group of formic acid or acetic acid, the ammonium carboxylate of formula (I) is based on formic acid or acetic acid and it can be prepared from such an acid or its salt.

Organic ammonium carboxylates used for controlling mineral dust are based on lower fatty acids.

As mentioned above, the ammonium ion of formula (I) may be primary (RNH₃⁺), secondary (R₂NH₂⁺), tertiary (R₃NH⁺) or quaternary (R₄N. Typical ammonium ions containing unsubstituted alkyls have been formed from water-soluble amines such as methylamine (g), dimethylamine, trimethylamine, ethylamine, diethylamine, etc.

Ammonium ions containing substituted alkyls have typically been formed from water-soluble amines, whose alkyl(s) may have been substituted with one or more hydroxyl groups. In formula (1), R₁ is preferably hydrogen and R₂ and R₃ have been selected from the group comprising hydrogen and methyl. R₄ is C₁-C₄-alkyl substituted with a hydroxyl group.

Organic ammonium carboxylates formed of lower alkanolamines are hence particularly useful. Among lower alkanolamines we may cite monoethanolamine. Prefrable aqueous solutions of ammonium carboxylates of formula (I) contain formic acid, acetic acid and monoethanolamine or trimethylmonoethanolamine. Trimethylmonoethanolamine is also called as acetylcholine.

One important group of useful alkanolamines comprises lower alkyl alkanolamines, such as methyl ethanolamine, dimethylethanolamine. Additional information about useful alkanolamines can be found in the book Kirk-Othmer, Encyclopedia of Chemical Technology 3rd Ed., Vol. 1, p. 944, which is incorporated in this disclosure.

It is particularly recommendable that R₁ is hydrogen, R₂ and R₃ are selected from the group comprising of hydrogen and methyl and R₄ is ethyl substituted with a hydroxyl group, preferably 2-hydroxy ethyl. In the most advantageous embodiment, the organic ammonium carboxylate of formula (I) is selected from the group comprising of a salt or a complex of formic acid or acetic acid and monoethanolamine or trimethylethanolamine.

In the practice, ammonium carboxylate of formula (I) is prepared e.g. by mixing an ammonium cation source and a carboxyl anion source in the desired molar ratio, either without a medium or by using an appropriate solvent such as water as a medium. When the starting materials are an amine and an acid, they are simply mixed during gentle heating, if necessary. When the starting materials consist of salts, they are typically dissolved separately in water, and then the solutions are combined. If a salt or a complex thus formed is hydrophobic, it will separate from the water phase as an unctuous or paste-like deposit or a wax-like precipitate, and it can be separated from the water phase by any known methods. When both the starting materials and the formed product are hydrophobic, the preparation can be carried out in an organic solvent instead of water. The freezing point depressant composition used in the invention comprises either fluid composing of ammonium carboxylate of formula (I) without solvent or ammonium carboxylate of formula (I) with appropriate solvent. Preferably solvent is an aqueous solution or a dispersion. Chemical stability: Preliminary results indicate that for instance a fluid pair: ethylene amide - formic acid could under special circumstances react and form amid when no solvent is present. Increasing the temperature favours amid formation. Nearly no esters are formed.

To be exact the invention relates the method defined in claim 1 or a combination defined in claim 21 comprising of moist or drops of aqueous solution of organic ammonium carboxylate and fine material.

In the method according to present invention it is sprayed aqueous solution of organic ammonium carboxylate of formula (I):

[NR¹R²R³R⁴]⁺ [R⁵(COO)], (I),

in which R¹, R², and R³ are selected from the group composing of hydrogen and methyl, R⁴ is a C₁-C₄-alkyl substituted with a hydroxyl group, R⁵ is hydrogen or methyl,
as a mist or drops in preventing dusting of fine material and in lowering the freezing point of said aqueous solution on the surface of said fine material ,
wherein said fine material is selected from the group composing of sand, crushed stone, crushed expanded clay, or crushed expanded clay aggregate, crushed cement or concrete, and chopped organic material.
in which method the concentration of ammonium carboxylate, in the aqueous solution of organic ammonium carboxylate of formula (I), which will be sprayed onto fine material is- in the range of 1 -50 %wt,
wherein either
a) said aqueous ammonium carboxylate solution of formula (I) is applied as a mist onto the surface of fine material in a case the average particle size of said fine material is 0,125 -0,025 mm (125 -25 um) wherein said mist have average particle size under 1.5 times of average particle size of said fine material or
b) said aqueous ammonium carboxylate solution of formula (I) is applied as drops onto the surface of said fine material in a case the average particle size of fine material is over 0,4 mm (400 um).

The method defined in claim 1, usually comprises also controlling the hydrophobicity and the hydrophilicity of the fine material which control is based on the concentration of ammonium carboxylate in aqueous solution of formula (I).

Preferable the concentration of ammonium carboxylate in the aqueous solution of formula (I) to be sprayed onto fine material is in the range of 1 -50 %wt, depending on the water content of the fine material.

More preferably the concentration of organic ammonium carboxylate in the aqueous solution of formula I is in the range of 1-7% wt-%, still more preferably in the range of 2-5 wt-%.

Prefereble the concentration of organic ammonium carboxylate of formula I which is then present on the surface of fine material or dust particles obtained from said fine material present is in the range of 1-7% wt-%, preferably in the range of 2-5 wt-%.The freezing point of the aqueous solution of organic ammonium carboxylate of formula (I) is then in the range of -5 to -50°C.
formed from said fine material selected from the group composing of sand, expanded clay, crushed stone, stone powder, crushed concrete, concrete powder, chopped organic material, minerals and metal powder and in lowering the freezing point of said aqueous solution.

One aspects, which is not according to the present invention relate to the use of aqueous solution of organic ammonium carboxylate of formula (I)

[NR¹R²R³R⁴]⁺ₙ [R⁵(COO)]⁻ⁿ, (I),

in which R¹, R², and R³ are selected from the group composing of hydrogen and methyl, R⁴ is a C₁-C₄-alkyl substituted with a hydroxyl group, R⁵ is hydrogen or methyl and n is 1,
as a solution in immersing fine material into said solution, wherein said fine material is selected from the group composing of sand, crushed stone, expanded clay, expanded clay aggregate (LECA), crushed cement or concrete, chopped organic material and minerals in lowering the freezing point of said aqueous solution.

In the combination according to present invention according to claim 21 there exists droplets of aqueous solution of organic ammonium carboxylate of formula (I) ):
In a combination of mist or drops of aqueous solution of organic ammonium carboxylate of formula (I) and fine material defined in claim 21:

[NR¹R²R³R⁴]⁺ [R⁵(COO)], (I),

in which R¹, R², and R³ are selected from the group composing of hydrogen and methyl, R⁴ is a C₁-C₄-alkyl substituted with a hydroxyl group, R⁵ is hydrogen or methyl,
and said fine material is selected from the group composing of sand, crushed stone, crushed expanded clay, or crushed expanded clay aggregate, crushed cement or concrete, and chopped organic material, ,
wherein said aqueous solution of organic ammonium carboxylate of formula (I) have been sprayed as a mist or drops onto the surface of said fine material particles, wherein
   a) in a case the average particle size of fine material is 0,125 -0,025 mm (125 - 25 um), the aqueous ammonium carboxylate solution of formula (I) has been applied as a mist onto the surface of fine material, which mist has an average particle size under 1.5 times of average particle size of said fine material or
   b) in a case the average particle size of fine material is over 0,4 mm (400 um) the aqueous ammonium carboxylate solution of formula (I) has been applied as drops onto the surface of said fine material. Preferably the ammonium carboxylate solution to be sprayed as a mist is an aqueous solution containing 1 -10 % w/w preferable 2 -5 % w/w of ammonium carboxylate, for example 3 -4 % ammonium carboxylate in water. However, this amount may be modified on the basis of moisture content of the fine material or dust particles originated on this dust particles. The exact concentration of ammonium carboxylate, present on the surface of fine material or dust cannot be measured exactly because water is evaporating continuously from the surface of fine material or dust.

In a case fine material already exists as a coarse dust it, which tends to form an aerosol with the surrounding air, it has usually a negative charge. Typical coarse dust is mineral dust (for example zink or nickel dust), cement or concrete dust, sand dust, stone dust, organic material dust (for example peat dust or wood dust. By using aqueous ammonium carboxylate solution as a fine mist, the negative charge of coarse dust particles can be neutralized and/or converted due to effect of positive charged ammonium ion to dust particles. By using mist of aqueous ammonium carboxylate solution neutralized dust particles from air will then be settled down onto ground.

The average particle size of coarse dust which is treated with the mist of aqueous ammonium carboxylate solution is so called coarse dust P10. Coarse dust P10 has an average particle size typically between 0,125 -0,025 mm (125 -25 um) and most of particles have a diameter over 10 um (over 0,010 mm). The mist of aqueous ammonium carboxylate solution should have particle size under 1.5 times of average particle size of coarse dust. More preferable the average particle size of aqueous ammonium carboxylate solution mist is about the same as the average particle size of coarse dust.

Mist is understood herein to mean droplets or drops having average diameter under 40 um (under 0,04 mm).

The neutralized coarse dust has then lowered freezing point because an aqueous ammonium carboxylate mist will act as an a freezing point depressant on a surface neutralized dust particles.

In a case most fine material (sand, crushed stone, crushed concrete, crushed cement minerals, chopped organic material, metal powder etc.) have mostly a bigger particle size than coarse dust, the aqueous solution of organic ammonium carboxylate can also be applied using other application methods, than spraying as a mist. Possible application methods for aqueous solution of organic ammonium carboxylate are: spraying aqueous ammonium carboxylate solution as drops onto fine material, immersion of fine material into ammonium carboxylate solution.

The aqueous ammonium carboxylate solution drops are understood here to have average diameter of 40 -500 um (0,4 -5 mm).

The aqueous solution of organic ammonium carboxylate (3 -5 % solution) have a freezing point from -3 °C to -15 °C preferably -from 5 to -10 °C. However, after applied the solution as a mist or drops onto surface of fine material (for example crushed stone) the water will evaporate from said aqueous solution. This will automatically lower the freezing point of remaining water in said combination of water and organic ammonium carboxylate: as can be seen later from (table IV) freezing point of aqueous solution of organic ammonium carboxylate of formula (I) will be considerably lowered when water leaves and rest of fluid comes more concentrated.

In one preferably use, aqueous solution of organic ammonium carboxylate of formula (I) is used as a mist or drops 1.0 - 2.0 l per 1000 kg of crushed stone, preferably 1.2 -1.5 l per 1000 kg of crushed stone.

One aspect of use, which is not according to present invention relate to immersion of fine material into aqueous solution of organic ammonium carboxylate of formula (I):

[NR¹R²R³R⁴]⁺ₙ [R⁵(COO)]⁻ⁿ, (I),

in which R¹, R², and R³ are selected from the group composing of hydrogen and methyl, R⁴ is a C₁-C₄-alkyl substituted with a hydroxyl group, R⁵ is hydrogen or methyl and n is 1. Fine material is selected from the group composing of sand, crushed stone, expanded clay, expanded clay aggregate (LECA), crushed cement or concrete, chopped organic material and minerals in lowering the freezing point of said aqueous solution.

Advantageously said fine material is crushed expanded clay, crushed expanded clay aggregate, crushed peat, zink powder, nickel powder, crushed cement or crushed concrete.

In the combination according to present invention according to claim 21 there exists droplets of aqueous solution of organic ammonium carboxylate of formula (I) ):
The aqueous solution of organic ammonium carboxylate is compatible with an ammonium salts of C₁-C₆ monocarboxylic acids or carboxylates of urea or ethylene glycol or propylene glycol, or glycerol or a mixture thereof. The ammonium carboxylate of formula (I) can be used with an ammonium salts of C₁-C₆ monocarboxylic acids or urea or ethylene glycol or propylene glycol, or glycerol or a mixture thereof carboxylates. By using aqueous solution of organic ammonium carboxylate with freezing point depressant composition with urea for de-icing or anti icing applications one can lower oxygen demand on the environment.

The aqueous solution of organic ammonium carboxylate of formula (I) may contain auxiliary substances as well. Typical auxiliary substances comprise such as additional corrosion inhibitors, biocides, coloring agents, surfactants, and viscosity intensifiers. The concentration of auxiliary substances will be in the range of 0.001 to 10 wt-% from the total weight of aqueous solution.

In a preferred embodiment of the invention the aqueous solution of organic ammonium carboxylate of formula (I) has concentration of sodium, potassium, calcium and magnesium 0 M. This means that there will be no chlorides formation from these cations after said aqueous solution have been applied for controlling dusting in mines.

Once applied onto surface of above defined fine material, drying of said combination will lead on the one hand to water loss and on the other hand absorbing of water by way of the organic ammonium carboxylate of formula (I) resulting in keeping the surface of said fine material moist. Actually, organic ammonium carboxylate of formula (I) will absorb water after water content of said aqueous solution have dropped at or below 15 wt-%. This is a very important aspect, because it will reduce drastically the amount of aqueous solution of organic ammonium carboxylate of formula (I) needed to keep dusting in control after applied onto fine material surface.

Biodegradativity of aqueous solutions used in the method of the invention for controlling dusting is relatively low: droplets of aqueous solution of organic ammonium carboxylate of formula (I), and fine material, wherein carboxylate originate from acetic acid, in form of fluid have BOD (biological oxygen demand) of 0.64 mg of O₂ at 20 °C for liter of said fluid in 5 days. COD (chemical oxygen demand) for the same combination 0.64 mg O₂ at 20 °C for liter of said fluid in 5 days.

Most closest prior art to above mentioned invention has been presented in the patent document WO 2011/089322. This document discloses aqueous solution of organic ammonium carboxylate which is preferably a salt of formic acid and monoethanolamine or a salt of lactic acid and monoethanolamine, triethanolaminen or a salt of lactic acid and monoethanolaminen and/or triethanolamine, Ammonium carboxylate part is a mixture of a salt of formic acid or lactic acid and monoethanolaminen. WO 2011/089322 discloses dust control but fails mentions that solution would be applied onto specific fine material as drops or mists having specific average size, depending average particle sizeof fine material.

### DESCRIPTION OF PREFERRED EMBODIMENTS OF THE INVENTION

The invention is described below in more greater details with the help of examples. Person skilled in the art will recognize that the properties of the compositions studied are such that they will make ideal mineral dust control aqueous solutions having also freezing point depressant properties for binding dusting of streets, ore mines, horse tracks etc.

In the following non-restricting examples we have presented some specific applications and properties of aqueous solutions comprising organic ammonium carboxylate of formula (I) as well as combinations comprising aqueous solution of organic ammonium carboxylate of formula (I) and fine material selected from the group composing of sand, crushed stone, minerals and metal powder.

The examples 5A, 5B, 6 and 7 are not according to present invention but illustration purposes only.

### Example 1

An ionogenic solution for controlling mineral dust formation was prepared by mixing 1 mole of formic acid (99%) with 1 mole of monoethanolamine (99%). Distilled water was added to the fluid mixture in order to made 3- 5 % by weight aqueous solution.

The freezing point of the solution was below -5°C, the electrical conductivity of the fluid was 61 mS/cm at 26°C, and pH of the fluid was 7.55 (measured directly from the solution).

### Example 2

An aqueous solutions was prepared by mixing 1 mole of formic acid (99%) with 1 mole of monoethanolamine (99%). Distilled water was added to the fluid mixture in order to made 3 - 5 by weight solution in water.

The freezing point of the solution was below -5°C, the Brookfield DV-I viscosity (20 rpm) was 10 mPas at -20°C, 10 mPas at -10°C, 10 mPas at 0°C, and Bohlin VOR viscosity (shear rate 23.1 1/s) was 4 mPas at 10°C, 3 mPas at 20°C, 2 mPas at 40°C, and 1.5 mPas at 60°C. The electrical conductivity of the fluid was 65 mS/cm at 26°C, and pH of the fluid was 7.54 (measured directly from the solution).

### Example 3A

Aqueous ammonium carboxylate solution mixture presented in examples 6 and 7 (below) was used in controlling dusting of crushed stone. This mixture comprises of ethanolamine in acetic acid and ethanolamine in formic acid (1:1). This mixture was sprayed as a 3 -5 % (w/w) aqueous solution in a form of mist or as drops onto surface of crushed stone and crushed stone dust. The sprayed mixture bind some moisture and prevented mineral dust and stone dust forming aerosol with surrounding air thus effectively preventing dusting by preventing forming charged dust particles. Additionally it lowered freezing of mineral dust and crushed stone/stone dust.

Crushed stone can be, for example, ore mineral originated from mining industry or rubble which have been gathered from town streets. Instead of above mentioned mixture comprising ethanolamine in acetic acid and ethanolamine in formic acid, one can also use aqueous ammonium carboxylate solution from example 2 (monoethanolamine and formic acid) or cholineamine in acetic acid or cholineamine in formic acid.

Committed peat material /peat dust and wood dust can also be treated in a similar way as crushed stone. Depending on the particle size of wood dust or committed peat / peat dust the aqueous ammonium carboxylate will be sprayed as a mist or as drops onto surface of peat/ peat dust or wood dust. Ammonium carboxylate was sprayed as a 3 -5 % (w/w) aqueous solution. The concentration present in the surface of committed peat material /peat dust and wood dust cannot be measured exactly because water is evaporating from the surface of fine material or dust..

### Example 4

Aqueous ammonium carboxylate solution mixture presented in examples 6 and 7 (below) was used in controlling dusting of crushed stone (street rubble) in situ. For this reason said aqueous ammonium carboxylate solution mixture was sprayed as drops onto pavement or city street. The concentration of ammonium carboxylate can be from 3 % w/w up to 85 %w/w calculated from the weight of the total aqueous ammonium carboxylate solution.

The sprayed mixture binded moisture and prevented crushed stone dust of forming gas-solid-particle-aerosol with surrounding air by neutralizing charged crushed stone dust particles. Additionally, this mixture lowered freezing point of crushed stone dust particles. This melts ice and snow which otherwise would have formed on crushed stone.

### Example 5A

Aqueous ammonium carboxylate solution mixture presented in examples 6 and 7 or example 2 was used in controlling dusting of crushed stone gathered from town street (street rubble): crushed stone was immersed into aqueous ammonium carboxylate solution (ammonium carboxylate concentration was 3 -5 % w/w). After this treatment the crushed stone can be reused.

### Example 5B

Aqueous ammonium carboxylate solution mixture presented in examples 6 and 7 or example 2 was used as a concentrated solution 50 % w/w or 85 %w/w by immersing crushed stone into said aqueous ammonium carboxylate solution (immersion solution). The actual concentration of immersion solution changed continuously during the immersion treatment because the fine material absorbs different amount ammonium carboxylate and water. Actual concentration of immersion solution varied from 5 % w/w to 85 %w/w.

After this treatment the crushed stone was applied onto frozen street. The ammonium carboxylate solution on the surface of crushed stone melted the ice and was diluted by means of melted water. After freezing crushed stone formed an excellent grit onto frozen street.

Instead of crushed stone also expanded clay, or expanded clay aggregate can also be treated in a similar way. Expanded clay or expanded clay aggregate can be applied onto pavements in a similar way as crushed stone in city streets.

### Examples 6 and 7

Solutions in examples 6 and 7 have been made in the same way as presented in examples 1-2, that is, by mixing 1 mole of an ammonium cation source and 1 mole of a carboxyl anion source (unless otherwise shown) together for obtaining a concentrated fluid and then adding water to the concentrated fluid, for obtaining diluted solutions.

### Explanation of tables

### Table I

In table 1 **(see annex 1)** has been shown formation of possible precipitates from fluids and diluted solutions obtained from fluids. Temperature was 20-25°C.

### Table II

The fluid and solution samples from selected examples of table 1 (see annex 2) were subjected to chilling to a temperature of +4 °C and then to further cooling to a temperature of -20°C. In these temperatures the possible turbidity, precipitation of these samples was observed.

As can be seen from table I and table II water-based solutions of organic of ammonium carboxylates are clear solutions independent whether they are diluted or not. This means that when they are used in controlling mineral dust they have no tendency to salt out after sprayed onto surface of crushed stones, sand, soil or metal. Therefore, they do not interfere for example vehicle brakes or transport belts used in mining processes.

### Table III

In table III (see annex 3) it has been given results from viscosity measurements compositions of examples 6 and 7. Viscocity was measured with Bohlin method (bold numbers) at shear rate 23.1 1/s and with Brookefield method (normal numbers) at shear rate 20 rpm. Additionally electrical conductivity, Ph and redox potential was measured for these compositions comprising fluids and solutions prepared from these fluids by adding distilled water.

As can be seen from table 3 the viscosity of compositions varies considerably depending on the quality of the fluid in a composition and fluid - solvent proportion (w/w). No solid crystals will be formed for instance if one uses combination ethanol amine / formic acid. Avoiding solid crystals is also a beneficial property for instance for an aqueous solution used for dust control.

### Table IVA

In table IV (see annex 4) it has been given the freezing points of aqueous solutions of organic ammonium carboxylate of formula (I), wherein R⁵ = H (formiate):

### Table IVB.

The freezing points of selected aqueous solutions of organic ammonium carboxylate of formula (I), wherein R⁵ = H (formiate) is given in table 4B (see annex 5).. In table IVB it has been given samples of organic ammonium carboxylate of formula (I) in water and their freezing points :
As can be seen form tables 4A and 4B (see annexes 4 and 5) as the concentration of water solutions of organic ammonium carboxy late of formula (I) increases it will readily lead to lower freezing points. For example those aqueous solutions of organic ammonium carboxylate of formula (I) presented in tables 4A and 4B having concentration about 10 wt -% have a freezing point about -5 °C. However when the concentration of organic ammonium carboxylate of formula (I) in the aqueuos solution increases, the freezing point of the aqueous solutions falls considerably , for example when the concentration of aqueuos solution is 30 (wt-%) the freezing point of said aqueous solution is about -20 °C. When the concentration of aqueuos solution is 60 wt-%) the freezing point of said aqueous solution is about -85 °C. The freezing point of -5 °C corresponds the freezing point of aqueous solutions of organic ammonium carboxylate of formula (I) which is ready-to -use ( 1-7 wt % aqueuos solution ). The freezing point of -30 C and -85 % corresponds the freezing point of the aqueous solution of organic ammonium carboxylate of formula (I) which have been sprayed onto surface of fine material when water has been evaporated.

### Annex 1

**Table I formation of possible precipitates from fluids and diluted solutions obtained from fluids. Temperature was 20-25°C.**

| | fluid Wt-% from solution | 100 | 90 | 80 | 60 | 40 | 20 | 5 | |
|---|---|---|---|---|---|---|---|---|---|
| | | | | | | | | | pH of 2% solution |
| Code/ex | fluid ethanolamine / | | | | | | | | |
| EAE /6 | acetic acid | Clear | Clear | Clear | Clear | Clear | Clear | Clear | 6.8 |
| EAM /7 | ethanolamine / formic acid | Clear | Clear | Clear | Clear | Clear | Clear | Clear | 3.7 |

### Annex 2

**Table II..**

| | Temperature +4 C | 100 | 90 | 80 | 60 | 40 | 20 | 5 |
|---|---|---|---|---|---|---|---|---|
| ex | | | | | | | | |
| 6 | ethanolamine / acetic acid | Clear | Clear | Clear | Clear | Clear | Clear | Clear |
| 7 | ethanolamine / formic acid | Clear | Clear | Clear | Clear | Clear | Clear | Clear |

| ex | Temperature -20°C | 100 | 90 | 80 | 60 | 40 | 20 | 5 |
|---|---|---|---|---|---|---|---|---|
| | | | | | | | | |
| 6 | ethanolamine / acetic acid | Clear/ liquid state | Clear/ liquid state | Clear/ liquid state | Clear/ liquid state | Clear/ liquid state | frozen | frozen |
| 7 | ethanolamine / formic acid | Clear/ liquid state | Clear/ liquid state | Clear/ liquid state | Clear/ liquid state | frozen | frozen | frozen |

### Annex 3

**Table III**

| | | | | | | | | |
|---|---|---|---|---|---|---|---|---|
| | fluid Wt-% | | | | | | | |
| monoethanolamine / acetic acid | from solution | 100 | 90 | 80 | 60 | 40 | 20 | 5 |
| water | water wt-% | 0 | 10 | 20 | 40 | 60 | 80 | 95 |

| | **°C** | **VISCOSITY mPas** | | | ***Bohlin VOR viscosity*** | | ***shear rate 23.1 1*/*s*** | |
|---|---|---|---|---|---|---|---|---|
| | | | | | Brookfield DV-I viscosity | | 20 rpm sp3 | |
| viscosity mPas / | -20 (repeat) | >20000 | >20000 | 12450 | 170 | 35 | X | X |
| | -20 | >20000 | 16740 | 1700 | 80 | 20 | X | X |
| | -10 | >20000 | 5150 | 700 | 60 | 15 | 10 | 5 |
| | 0 | 27850 | 2160 | 330 | 40 | 10 | 10 | 5 |
| | 10 | ***15250*** | ***1152*** | ***210*** | **23** | **6** | **2** | **1.7** |
| | **20** | **5665** | ***556*** | ***118*** | **15** | **5** | **2** | **1.3** |
| | **40** | ***1220*** | ***154*** | **41** | **8** | **3** | **1.5** | **1.1** |
| | **60** | ***345*** | **63** | **20** | **5** | **2** | **1** | **0.7** |
| conductivity mS /cm | | 0.534 | 2.24 | 7.1 | 25.9 | 46.9 | 47.8 | 20.2 |
| T °C | | 25.4 | 25.9 | 26 | 25.6 | 25.4 | 25.1 | 24.9 |
| pH °C 22 | | 7.96 | 7.81 | 7.68 | 7.34 | 7.07 | 6.87 | 6.79 |
| REDOX | | +31 | +54 | +69 | +107 | +146 | +179 | +216 |

**Table III (continued)**

| | | | | | | | | |
|---|---|---|---|---|---|---|---|---|
| | fluid Wt-% | | | | | | | |
| Composition: monoethanolamine / formic acid | from solution | 100 | 90 | 80 | 60 | 40 | 20 | 5 |
| water | water wt-% | 0 | 10 | 20 | 40 | 60 | 80 | 95 |
| | pale oily light liquid | | | | | | | |

| | °C | **VISCOSITY mPas** | | | ***Bohlin VOR viscosity*** | | ***shear rate 23.1 1*/*s*** | |
|---|---|---|---|---|---|---|---|---|
| | | | | | Brookfield DV-I viscosity | | 20 rpm sp3 | |
| viscosity mPas / | -30 | | | | | | | |
| | -20 | 4350 | 680 | 230 | 30 | 10 | X | X |
| | -10 | 2830 | 410 | 130 | 20 | 10 | 5 | X |
| | 0 | 1335 | 240 | 75 | 15 | 10 | 5 | 5 |
| | 10 | **646** | ***123*** | **41** | **9** | **4** | **2** | **1.5** |
| | 20 | ***325*** | **72** | **26** | **6** | **3** | **1.7** | **1.2** |
| | 40 | ***119*** | **31** | **13** | **4** | **2** | **1.2** | ***0.95*** |
| | 60 | **47** | **17** | **7** | **3** | **1.5** | **1.1** | ***0.9*** |
| conductivity mS /cm | | 15.9 | 27.3 | 40.4 | 61 | 65 | 46.9 | 16 |
| T °C | | 26.1 | 25.9 | 25.8 | 25.6 | 25.5 | 25.5 | 25.8 |
| pH / 22°C | | 7.75 | 7.67 | 7.6 | 7.55 | 7.54 | 7.53 | 7.51 |
| REDOX potential | | -321 | -244 | -164 | -110 | -75 | -48 | +4 |

### Annex 4

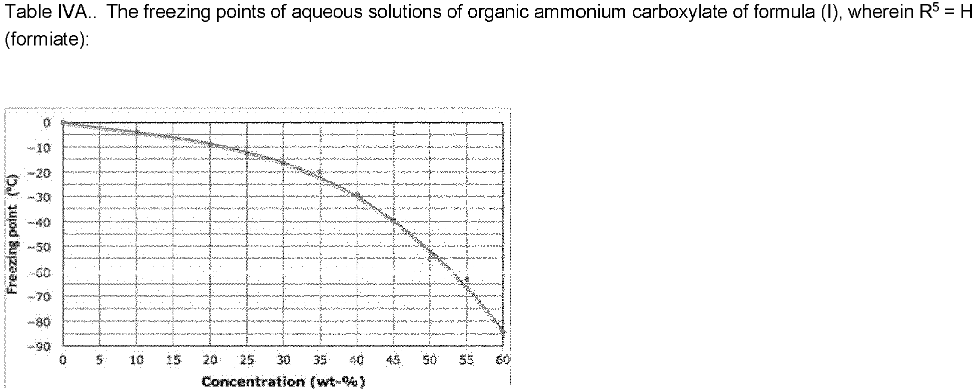

### Annex 5

**Table IVB . The freezing points of selected aqueous solutions of organic ammonium carboxylate of formula (I), wherein R⁵ = H (formiate) are given in table 4B.**

| **Sample** | **Concentration (wt-%)** | **Freezing point (°C)** |
|---|---|---|
| Water | 0 | 0 |
| HTF-20 | 20 | -8,96 |
| HTF-25 | 25 | -12,44 |
| HTF-30 | 30 | -16,51 |
| HTF-35 | 35 | -20,13 |
| HTF-40 | 40 | -29,33 |
| HTF-45 | 45 | -39,48 |
| HTF-50 | 50 | -54,95 |
| HTF-55 | 55 | -63,01 |
| HTF-60 | 60 | -84,50 |
| | | |

## Claims

1. The method for spraying an aqueous solution of organic ammonium carboxylate of formula (I):
[NR¹R²R³R⁴]⁺ [R⁵(COO)]⁻, (I),
in which R¹, R², and R³ are selected from the group composing of hydrogen and methyl, R⁴ is a C₁-C₄-alkyl substituted with a hydroxyl group, R⁵ is hydrogen or methyl;
as a mist or drops onto fine material to prevent dusting of fine and to lower the freezing point of said aqueous solution of organic ammonium carboxylate of formula (I): after said solution of aqueous organic ammonium carboxylate of formula (I):has been sprayed on the surface of said fine material;
wherein said fine material is selected from the group composing of sand, crushed stone, crushed expanded clay, or crushed expanded clay aggregate, crushed cement or concrete and chopped organic material,
in which method the concentration of ammonium carboxylate, in the aqueous solution of organic ammonium carboxylate of formula (I), which will be sprayed onto fine material is- in the range of 1 -50 %wt,
wherein either
a) said aqueous ammonium carboxylate solution of formula (I) is applied as a mist onto the surface of fine material in a case the average particle size of said fine material is 0,125 -0,025 mm (125 -25 um) wherein said mist have average particle size under 1.5 times of average particle size of said fine material or
b) said aqueous ammonium carboxylate solution of formula (I) is applied as drops onto the surface of said fine material in a case the average particle size of fine material is over 0,4 mm (400 um).

2. The method defined in claim 1, which additionally comprises controlling the hydrophobicity and the hydrophilicity of the fine material which control is based on the concentration of ammonium carboxylate in aqueous solution of formula (I).

3. The method defined in claim 1, wherein the concentration of organic ammonium carboxylate in the aqueous solution of formula I is in the range of 1-10 % wt-%, preferably in the range of 2-5 wt-%.

4. The method defined in any of the previous claims, in which the freezing point of the aqueous solution of organic ammonium carboxylate of formula (I) is in the range of -5 to -50°C.

5. The method defined in any of the previous claims, wherein the surface of the fine material which have been treated with aqueous solution of organic ammonium carboxylate of formula (I) is hydrophilic.

6. The method defined in any of the previous claims, wherein said fine material is crushed expanded clay, crushed expanded clay aggregate, crushed peat, peat powder or chopped wood,.

7. The method defined in claim 1 , wherein preventing of dusting of fine material and in lowering the freezing point of said aqueous solution present on the surface of said fine material, by spraying said mist or drops onto fine ,additionally comprises controlling the hydrophobicity and the hydrophilicity of the fine material which control is based on the concentration of ammonium carboxylate in aqueous solution of formula (I).

8. The method defined in any of the previous claims 1 - 7, wherein R¹, R² and R³ are all hydrogen and R⁴ is an ethyl substituted with a hydroxyl group, preferably 2-hydroxy ethyl.

9. The method defined in any of the previous claims 1-8, wherein R¹, R² and R³ are all methyl and R⁴ is an ethyl substituted with a hydroxyl group, preferably 2-hydroxy ethyl.

10. The method defined in any of the previous claims 1-9, wherein the organic ammonium carboxylate of formula (I) is a salt of formic acid and monoethanolamine or a salt of acetic acid and monoethanolamine.

11. The method defined in any of the previous claims 1-10, wherein the aqueous solution of organic ammonium carboxylate of formula (I) contains organic ammonium carboxylate of formula (1) and water in a weight ratio in the range of 1:50-1: 15, preferably in the range of 1:33-1:20.

12. The method defined in any of the previous claims 1-11, wherein the aqueous solution of organic ammonium carboxylate of formula (I) contains distilled water only.

13. The method defined in claim any of the previous claims 1-12, wherein the concentration of sodium, potassium, calcium and magnesium is 0 M in the aqueous solution of organic ammonium carboxylate of formula (I).

14. The method as defined in any of previous claims 1-13, wherein the aqueous solution of organic ammonium carboxylate of formula (I) is additionally admixed with compounds selected from the group comprising glycols, preferably ethylene glycol or propylene glycol, glycerols and viscosity modifying polymers, so that said aqueous organic ammonium carboxylate composition contains 5 to 97.5 wt-% of water.

15. The method as defined in any of the previous claims 1-14, wherein aqueous solution of organic ammonium carboxylate of formula (I) is additionally admixed with auxiliary substances such as additional corrosion inhibitors, biocides, fragnance(s), coloring agents, surfactants, and viscosity intensifiers, so that the concentration of auxiliary substances will be in the range of 0.001 to 10 wt-% from the total weight of aqueous solution.

16. The method defined in any of previous claims 1-15, wherein the aqueous solution of organic ammonium carboxylate of formula (I) contains formic acid, acetic acid and monoethanolamine or trimethylmonoethanolamine.

17. The method defined in any of the previous claims 1-16, wherein aqueous solution of organic ammonium carboxylate of formula (I) is used 1-2 l per 1000 kg of crushed stone, preferably 1.2 -1.5 l per 1000 kg of crushed stone.

18. The method defined in any of the previous claims 1-17, wherein freezing point of said aqueous solution of organic ammonium carboxylate of formula (I) is from -3 °C to -15 °C preferably from -5 °C to -10 °C.

19. The method defined in claim 1, further including a step wherein the solution comprising aqueous organic ammonium carboxylate of formula (I) is prepared by mixing, in situ, an aqueous solution of 1- 5 wt-% of amine with an aqueous solution of 1- 5 wt-% of acid.

20. The method defined in claim 1 in which drops of aqueous ammonium carboxylate solution drops to have average diameter of 40 -500 um (0,4 -5 mm).

21. A combination of mist or drops of an aqueous solution of organic ammonium carboxylate of formula (I) and fine material which combination has been prepared by the method defined in claim 1
and in which combination the aqueous solution of organic ammonium carboxylate is of the formula (I):
[NR¹R²R³R⁴]⁺ [R⁵(COO)]⁻, (I),
in which R¹, R², and R³ are selected from the group composing of hydrogen and methyl, R⁴ is a C₁-C₄-alkyl substituted with a hydroxyl group, R⁵ is hydrogen or methyl wherein the concentration of ammonium carboxylate, in the aqueous solution of organic ammonium carboxylate of formula (I), which will be sprayed onto fine material is- in the range of 1 -50 %wt, and
fine material is selected from the group composing of sand, crushed stone, crushed expanded clay, or crushed expanded clay aggregate, crushed cement or concrete and chopped organic material, wherein
said aqueous solution of organic ammonium carboxylate of formula (I) have been sprayed as a mist or drops onto the surface of said fine material particles, and
a) in a case the average particle size of fine material is 0,125 -0,025 mm (125 - 25 um), the aqueous ammonium carboxylate solution of formula (I) has been applied as a mist onto the surface of fine material, which mist has an average particle size under 1.5 times of average particle size of said fine material or
b) in a case the average particle size of fine material is over 0,4 mm (400 um) the aqueous ammonium carboxylate solution of formula (I) has been applied as drops onto the surface of said fine material..

22. The combination defined in claim 21 comprising drops of aqueous solution of organic ammonium carboxylate of formula (I), and fine material, wherein carboxylate originate from acetic acid, in form of fluid and having BOD (biological oxygen demand) of 0.64 mg of O₂ at 20 °C for liter of said fluid in 5 days.

23. The combination defined in claim 21 comprising drops of aqueous solution of organic ammonium carboxylate of formula (I), and fine material, wherein carboxylate originate from acetic acid, in form of fluid, and having COD (chemical oxygen demand) of 0.64 mg O₂ at 20 °C for liter of said fluid in 5 days.

## Patentansprüche

1. Verfahren zum Versprühen einer wässrigen Lösung von organischem Ammoniumcarboxylat der Formel (I):
[NR¹R²R³R⁴]⁺[R⁵(COO)]⁻, (I),
in der R¹, R² und R³ ausgewählt sind aus der Gruppe, bestehend aus Wasserstoff und Methyl, R⁴ ein C₁-C₄-Alkyl ist, das mit einer Hydroxylgruppe substituiert ist, R⁵ Wasserstoff oder Methyl ist;
als Nebel oder Tropfen auf Feinmaterial, um ein Verstauben des Feinmaterials zu verhindern und den Gefrierpunkt der wässrigen Lösung des organischen Ammoniumcarboxylats der Formel (I): zu senken, nachdem die Lösung des wässrigen organischen Ammoniumcarboxylats der Formel (I): auf die Oberfläche des Feinmaterials gesprüht wurde;
wobei das Feinmaterial aus der Gruppe ausgewählt wird, bestehend aus Sand, gebrochenem Stein, gebrochenem Blähton oder gebrochenem Blähtonzuschlag, gebrochenem Zement oder Beton und gehacktem organischem Material,
in dem Verfahren die Konzentration von Ammoniumcarboxylat in der wässrigen Lösung von organischem Ammoniumcarboxylat der Formel (I), die auf feines Material gesprüht wird, im Bereich von 1-50 Gew.-% liegt,
wobei entweder
a) die wässrige Ammoniumcarboxylatlösung der Formel (I) als Nebel auf die Oberfläche des Feinmaterials aufgebracht wird, wobei die durchschnittliche Partikelgröße des Feinmaterials 0,125-0,025 mm (125-25 um) beträgt, wobei der Nebel eine durchschnittliche Partikelgröße unter dem 1,5-fachen der durchschnittlichen Partikelgröße des Feinmaterials aufweist, oder
b) die wässrige Ammoniumcarboxylatlösung der Formel (I) als Tropfen auf die Oberfläche des Feinmaterials aufgetragen wird, wenn die durchschnittliche Partikelgröße des Feinmaterials über 0,4 mm (400 um) liegt.

2. Verfahren nach Anspruch 1, das zusätzlich das Steuern der Hydrophobie und der Hydrophilie des Feinmaterials umfasst, wobei die Steuerung auf der Konzentration von Ammoniumcarboxylat in wässriger Lösung der Formel (I) basiert.

3. Verfahren nach Anspruch 1, wobei die Konzentration des organischen Ammoniumcarboxylats in der wässrigen Lösung der Formel I im Bereich von 1-10 Gew.-%, vorzugsweise im Bereich von 2-5 Gew.-% liegt.

4. Verfahren nach einem der vorstehenden Ansprüche, bei dem der Gefrierpunkt der wässrigen Lösung des organischen Ammoniumcarboxylats der Formel (I) im Bereich von -5 bis -50 °C liegt.

5. Verfahren nach einem der vorstehenden Ansprüche, wobei die Oberfläche des Feinmaterials, das mit einer wässrigen Lösung eines organischen Ammoniumcarboxylats der Formel (I) behandelt wurde, hydrophil ist.

6. Verfahren nach einem der vorstehenden Ansprüche, wobei es sich bei dem Feinmaterial um zerkleinerten Blähton, zerkleinerten Blähtonzuschlag, zerkleinerten Torf, Torfpulver oder gehacktes Holz handelt.

7. Verfahren nach Anspruch 1, wobei das Verhindern des Verstaubens von Feinmaterial und das Senken des Gefrierpunkts der wässrigen Lösung, die auf der Oberfläche des Feinmaterials vorhanden ist, durch Sprühen des Nebels oder der Tropfen auf Feinmaterial zusätzlich das Steuern der Hydrophobizität und der Hydrophilizität des Feinmaterials umfasst, wobei die Steuerung auf der Konzentration von Ammoniumcarboxylat in der wässrigen Lösung der Formel (I) basiert.

8. Verfahren nach einem der vorstehenden Ansprüche 1 bis 7, wobei R¹, R² und R³ alle Wasserstoff sind und R⁴ ein mit einer Hydroxylgruppe substituiertes Ethyl, vorzugsweise 2-Hydroxyethyl, ist.

9. Verfahren nach einem der vorstehenden Ansprüche 1 bis 8, wobei R¹, R² und R³ alle Methyl sind und R⁴ ein mit einer Hydroxylgruppe substituiertes Ethyl, vorzugsweise 2-Hydroxyethyl, ist.

10. Verfahren nach einem der vorstehenden Ansprüche 1 bis 9, wobei das organische Ammoniumcarboxylat der Formel (I) ein Salz von Ameisensäure und Monoethanolamin oder ein Salz von Essigsäure und Monoethanolamin ist.

11. Verfahren nach einem der vorstehenden Ansprüche 1 bis 10, wobei die wässrige Lösung von organischem Ammoniumcarboxylat der Formel (I) organisches Ammoniumcarboxylat der Formel (1) und Wasser in einem Gewichtsverhältnis im Bereich von 1:50 bis 1:15, vorzugsweise im Bereich von 1:33 bis 1:20, enthält.

12. Verfahren nach einem der vorstehenden Ansprüche 1 bis 11, wobei die wässrige Lösung des organischen Ammoniumcarboxylats der Formel (I) nur destilliertes Wasser enthält.

13. Verfahren nach einem der vorstehenden Ansprüche 1 bis 12, wobei die Konzentration von Natrium, Kalium, Calcium und Magnesium 0 M in der wässrigen Lösung des organischen Ammoniumcarboxylats der Formel (I) beträgt.

14. Verfahren nach einem der vorstehenden Ansprüche 1 bis 13, wobei die wässrige Lösung des organischen Ammoniumcarboxylats der Formel (I) zusätzlich mit Verbindungen vermischt wird, die aus der Gruppe ausgewählt sind, bestehend aus Glykolen, vorzugsweise Ethylenglykol oder Propylenglykol, Glycerinen und viskositätsmodifizierenden Polymeren, sodass die wässrige organische Ammoniumcarboxylatzusammensetzung 5 bis 97,5 Gew.-% Wasser enthält.

15. Verfahren nach einem der vorstehenden Ansprüche 1 bis 14, wobei die wässrige Lösung des organischen Ammoniumcarboxylats der Formel (I) zusätzlich mit Hilfsstoffen wie zusätzlichen Korrosionsinhibitoren, Bioziden, Duftstoffen, Farbmitteln, Tensiden und Viskositätsverstärkern versetzt wird, sodass die Konzentration der Hilfsstoffe im Bereich von 0,001 bis 10 Gew.-% bezogen auf das Gesamtgewicht der wässrigen Lösung liegt.

16. Verfahren nach einem der vorstehenden Ansprüche 1 bis 15, wobei die wässrige Lösung des organischen Ammoniumcarboxylats der Formel (I) Ameisensäure, Essigsäure und Monoethanolamin oder Trimethylmonoethanolamin enthält.

17. Verfahren nach einem der vorstehenden Ansprüche 1 bis 16, wobei 1-2 l pro 1000 kg gebrochenem Gestein, vorzugsweise 1,2-1,5 l pro 1000 kg gebrochenem Gestein, als wässrige Lösung von organischem Ammoniumcarboxylat der Formel (I) verwendet werden.

18. Verfahren nach einem der vorstehenden Ansprüche 1 bis 17, wobei der Gefrierpunkt der wässrigen Lösung des organischen Ammoniumcarboxylats der Formel (I) von -3 °C bis -15 °C, vorzugsweise von -5 °C bis -10 °C, beträgt.

19. Verfahren nach Anspruch 1, ferner einschließend einen Schritt, wobei die Lösung, die wässriges organisches Ammoniumcarboxylat der Formel (I) umfasst, durch Mischen einer wässrigen Lösung von 1-5 Gew.-% Amin mit einer wässrigen Lösung von 1-5 Gew.-% Säure in situ hergestellt wird.

20. Verfahren nach Anspruch 1, bei dem die Tropfen der wässrigen Ammoniumcarboxylatlösung einen durchschnittlichen Durchmesser von 40-500 um (0,4-5 mm) aufweisen.

21. Kombination aus Nebel oder Tropfen einer wässrigen Lösung von organischem Ammoniumcarboxylat der Formel (I) und Feinmaterial, wobei die Kombination nach dem in Anspruch 1
definierten Verfahren hergestellt wurde und wobei in der Kombination die wässrige Lösung von organischem Ammoniumcarboxylat die Formel (I) aufweist:
[NR¹R²R³R⁴]⁺[R⁵(COO)]⁻, (I),
in der R¹, R² und R³ ausgewählt sind aus der Gruppe, bestehend aus Wasserstoff und Methyl, R⁴ ein C₁-C₄-Alkyl ist, das mit einer Hydroxylgruppe substituiert ist, R⁵ Wasserstoff oder Methyl ist, wobei die Konzentration von Ammoniumcarboxylat in der wässrigen Lösung von organischem Ammoniumcarboxylat der Formel (I), die auf Feinmaterial gesprüht wird, im Bereich von 1-50 Gew.-% liegt, und
das Feinmaterial aus der Gruppe ausgewählt wird, bestehend aus Sand, gebrochenem Stein, gebrochenem Blähton oder gebrochenem Blähtonzuschlag, gebrochenem Zement oder Beton und gehacktem organischem Material,
wobei
die wässrige Lösung des organischen Ammoniumcarboxylats der Formel (I) als Nebel oder Tropfen auf die Oberfläche der Feinmaterialpartikel gesprüht wurde, und
a) in einem Fall, in dem die durchschnittliche Partikelgröße des Feinmaterials 0,125-0,025 mm (125-25 um) beträgt, die wässrige Ammoniumcarboxylatlösung der Formel (I) als Nebel auf die Oberfläche des Feinmaterials aufgebracht wurde, wobei der Nebel eine durchschnittliche Partikelgröße aufweist, die unter dem 1,5-fachen der durchschnittlichen Partikelgröße des Feinmaterials liegt, oder
b) in einem Fall, in dem die durchschnittliche Partikelgröße des Feinmaterials über 0,4 mm (400 um) liegt, die wässrige Ammoniumcarboxylatlösung der Formel (I) in Form von Tropfen auf die Oberfläche des Feinmaterials aufgetragen wurde.

22. Kombination nach Anspruch 21, umfassend Tropfen einer wässrigen Lösung von organischem Ammoniumcarboxylat der Formel (I) und Feinmaterial, wobei das Carboxylat aus Essigsäure stammt, in Form eines Fluids und mit einem BSB (biologischen Sauerstoffbedarf) von 0,64 mg O₂ bei 20 °C pro Liter des Fluids in 5 Tagen.

23. Kombination nach Anspruch 21, umfassend Tropfen einer wässrigen Lösung von organischem Ammoniumcarboxylat der Formel (I) und Feinmaterial, wobei das Carboxylat aus Essigsäure stammt, in Form eines Fluids und mit einem CSB (chemischen Sauerstoffbedarf) von 0,64 mg O₂ bei 20 °C pro Liter des Fluids in 5 Tagen.

## Revendications

1. Procédé de pulvérisation d'une solution aqueuse de carboxylate d'ammonium organique de formule (I) :
[NR¹R²R³R⁴]⁺[R⁵(COO)]⁻, (I),
dans lequel R¹, R² et R³ sont choisis dans le groupe composé d'hydrogène et de méthyle, R⁴ est un alkyle en C₁-C₄ substitué par un groupe hydroxyle, R⁵ est hydrogène ou méthyle ;
sous forme d'un brouillard ou de gouttes sur un matériau fin pour empêcher le poudrage du matériau fin et pour abaisser le point de congélation de ladite solution aqueuse de carboxylate d'ammonium organique de formule (I) : après que ladite solution de carboxylate d'ammonium organique aqueux de formule (I) : ait été pulvérisée sur la surface dudit matériau fin ;
dans lequel ledit matériau fin est choisi dans le groupe composé de sable, de pierre concassée, d'argile expansée concassée ou de granulats d'argile expansée concassée, de ciment ou de béton concassé et de matière organique hachée,
dans lequel le procédé, la concentration de carboxylate d'ammonium, dans la solution aqueuse de carboxylate d'ammonium organique de formule (I), qui sera pulvérisée sur un matériau fin, est dans la plage de 1 à 50 % en poids,
dans lequel soit
a) ladite solution aqueuse de carboxylate d'ammonium de formule (I) est appliquée sous forme d'un brouillard sur la surface d'un matériau fin, dans le cas où la taille moyenne de particules dudit matériau fin est de 0,125 à 0,025 mm (125 à 25 µm), dans lequel ledit brouillard à une taille moyenne de particules inférieure à 1,5 fois la taille moyenne de particules dudit matériau fin soit
b) ladite solution aqueuse de carboxylate d'ammonium de formule (I) est appliquée sous forme de gouttes sur la surface dudit matériau fin dans le cas où la taille moyenne de particules du matériau fin est supérieure à 0,4 mm (400 µm).

2. Procédé selon la revendication 1, qui comprend en outre la commande de l'hydrophobie et de l'hydrophilie du matériau fin, laquelle commande est sur la base de la concentration de carboxylate d'ammonium dans une solution aqueuse de formule (I).

3. Procédé selon la revendication 1, dans lequel la concentration de carboxylate d'ammonium organique dans la solution aqueuse de formule I est dans la plage de 1 à 10 % en poids, de préférence dans la plage de 2 à 5 % en poids.

4. Procédé selon l'une quelconque des revendications précédentes, dans lequel le point de congélation de la solution aqueuse de carboxylate d'ammonium organique de formule (I) est dans la plage de -5 à -50 °C.

5. Procédé selon l'une quelconque des revendications précédentes, dans lequel la surface du matériau fin qui a été traité avec une solution aqueuse de carboxylate d'ammonium organique de formule (I) est hydrophile.

6. Procédé selon l'une quelconque des revendications précédentes, dans lequel ledit matériau fin est de l'argile expansée concassée, des granulats d'argile expansée concassée, de la tourbe concassée, de la poudre de tourbe ou du bois haché.

7. Procédé selon la revendication 1, dans lequel la prévention du poudrage du matériau fin et l'abaissement du point de congélation de ladite solution aqueuse présente à la surface dudit matériau fin, par pulvérisation dudit brouillard ou desdites gouttes sur le matériau fin, comprend en outre la commande de l'hydrophobie et de l'hydrophilie du matériau fin, laquelle commande est sur la base de la concentration de carboxylate d'ammonium dans la solution aqueuse de formule (I).

8. Procédé selon l'une quelconque des revendications précédentes 1 à 7, dans lequel R¹, R² et R³ sont tous de l'hydrogène et R⁴ est un éthyle substitué par un groupe hydroxyle, de préférence 2-hydroxyéthyle.

9. Procédé selon l'une quelconque des revendications précédentes 1 à 8, dans lequel R¹, R² et R³ sont tous un méthyle et R⁴ est un éthyle substitué par un groupe hydroxyle, de préférence 2-hydroxyéthyle.

10. Procédé selon l'une quelconque des revendications précédentes 1 à 9, dans lequel le carboxylate d'ammonium organique de formule (I) est un sel d'acide formique et de monoéthanolamine ou un sel d'acide acétique et de monoéthanolamine.

11. Procédé selon l'une quelconque des revendications précédentes 1 à 10, dans lequel la solution aqueuse de carboxylate d'ammonium organique de formule (I) contient du carboxylate d'ammonium organique de formule (1) et de l'eau dans un rapport pondéral dans la plage de 1:50 à 1:15, de préférence dans la plage de 1:33 à 1:20.

12. Procédé selon l'une quelconque des revendications précédentes 1 à 11, dans lequel la solution aqueuse de carboxylate d'ammonium organique de formule (I) contient uniquement de l'eau distillée.

13. Procédé selon l'une quelconque des revendications précédentes 1 à 12, dans lequel la concentration de sodium, de potassium, de calcium et de magnésium est de 0 M dans la solution aqueuse de carboxylate d'ammonium organique de formule (I).

14. Procédé selon l'une quelconque des revendications précédentes 1 à 13, dans lequel la solution aqueuse de carboxylate d'ammonium organique de formule (I) est en outre mélangée à des composés choisis dans le groupe comprenant des glycols, de préférence de l'éthylène glycol ou du propylène glycol, des glycérols et des polymères modificateurs de viscosité, de sorte que ladite composition aqueuse de carboxylate d'ammonium organique contienne de 5 à 97,5 % en poids d'eau.

15. Procédé selon l'une quelconque des revendications précédentes 1 à 14, dans lequel une solution aqueuse de carboxylate d'ammonium organique de formule (I) est en outre mélangée à des substances auxiliaires telles que des inhibiteurs de corrosion, des biocides, des parfums, des agents colorants, des tensioactifs et des intensificateurs de viscosité supplémentaires, de sorte que la concentration des substances auxiliaires soit dans la plage de 0,001 et 10 % en poids du poids total de la solution aqueuse.

16. Procédé selon l'une quelconque des revendications précédentes 1 à 15, dans lequel la solution aqueuse de carboxylate d'ammonium organique de formule (I) contient de l'acide formique, de l'acide acétique et de la monoéthanolamine ou de la triméthylmonoéthanolamine.

17. Procédé selon l'une quelconque des revendications précédentes 1 à 16, dans lequel une solution aqueuse de carboxylate d'ammonium organique de formule (I) est utilisée à raison de 1 à 2 l pour 1000 kg de pierre concassée, de préférence de 1,2 à 1,5 1 pour 1000 kg de pierre concassée.

18. Procédé selon l'une quelconque des revendications précédentes 1 à 17, dans lequel le point de congélation de ladite solution aqueuse de carboxylate d'ammonium organique de formule (I) est de -3 °C à -15 °C, de préférence de -5 °C à -10 °C.

19. Procédé selon la revendication 1, comportant en outre une étape dans laquelle la solution comprenant du carboxylate d'ammonium organique aqueux de formule (I) est préparée en mélangeant, in situ, une solution aqueuse de 1 à 5 % en poids d'amine avec une solution aqueuse de 1 à 5 % en poids d'acide.

20. Procédé selon la revendication 1, dans lequel les gouttes de solution aqueuse de carboxylate d'ammonium ont un diamètre moyen de 40 à 500 µm (0,4 à 5 mm).

21. Combinaison de brouillard ou de gouttelettes d'une solution aqueuse de carboxylate d'ammonium organique de formule (I) et de matériau fin, laquelle combinaison a été préparée selon le procédé selon la revendication 1 et dans laquelle la combinaison la solution aqueuse de carboxylate d'ammonium organique a la formule (I) :
[NR¹R²R³R⁴]⁺[R⁵ (COO)]⁻, (I),
dans laquelle R¹, R² et R³ sont choisis dans le groupe composé de l'hydrogène et du méthyle, R⁴ est un alkyle en C₁-C₄ substitué par un groupe hydroxyle, R⁵ est hydrogène ou méthyle, dans laquelle la concentration de carboxylate d'ammonium dans la solution aqueuse de carboxylate d'ammonium organique de formule (I), qui sera pulvérisée sur le matériau fin, est dans la plage de 1 à 50 % en poids, et
le matériau fin est choisi dans le groupe composé de sable, de pierre concassée, d'argile expansée concassée ou de granulats d'argile expansée concassée, de ciment ou de béton concassé et de matière organique hachée,
dans laquelle
ladite solution aqueuse de carboxylate d'ammonium organique de formule (I) a été pulvérisée sous forme d'un brouillard ou de gouttes sur la surface desdites particules de matériau fin, et
a) dans le cas où la taille moyenne de particules du matériau fin est de 0,125 à 0,025 mm (125 à 25 µm), une solution aqueuse de carboxylate d'ammonium de formule (I) a été appliquée sous forme d'un brouillard sur la surface du matériau fin, lequel brouillard a une taille moyenne de particules inférieure à 1,5 fois la taille moyenne de particules dudit matériau fin, ou
b) dans le cas où la taille moyenne de particules du matériau fin est supérieure à 0,4 mm (400 µm), la solution aqueuse de carboxylate d'ammonium de formule (I) a été appliquée sous forme de gouttes sur la surface dudit matériau fin.

22. Combinaison selon la revendication 21, comprenant des gouttes de solution aqueuse de carboxylate d'ammonium organique de formule (I) et de matériau fin, dans laquelle le carboxylate provient de l'acide acétique, sous forme de fluide et ayant une DBO (demande biologique en oxygène) de 0,64 mg d'O₂ à 20 °C par litre dudit fluide en 5 jours.

23. Combinaison selon la revendication 21, comprenant des gouttes de solution aqueuse de carboxylate d'ammonium organique de formule (I) et de matériau fin, dans laquelle le carboxylate provient de l'acide acétique, sous forme de fluide, et ayant une DCO (demande chimique en oxygène) de 0,64 mg d'O₂ à 20 °C par litre dudit fluide en 5 jours.
